# EUROPEAN PATENT APPLICATION

(11) **EP 4 559 327 A1**
(43) Date of publication of application: **28.05.2025**
(21) Application number: 23843064.9
(22) Date of filing: 21.07.2023
(51) Int. Cl.: A23L 33/13, A23L 5/00, A23L 33/125, A23L 33/135

(54) **ENTERIC CAPSULE FOR IMPROVING INTESTINAL BACTERIAL FLORA ENCAPSULATING NICOTINAMIDE MONONUCLEOTIDE**

(30) Priority: 21.07.2022 JP 2022116167
(71) Applicant: Tanaka, Megumi, Chigasaki-shi, Kanagawa 253-0054 (JP); Tanaka, Tsunemaru, Chigasaki-shi, Kanagawa 253-0054 (JP); Kitano, Hiroaki, Kawagoe-shi, Saitama 350-0035 (JP)
(72) Inventor: YACHIE Ayako, Tokyo 141-0022 (JP); GHOSH Samik, Tokyo 141-0022 (JP); TANAKA Megumi, Chigasaki-shi, Kanagawa 253-0054 (JP); TANAKA Tsunemaru, Chigasaki-shi, Kanagawa 253-0054 (JP); KITANO Hiroaki, Kawagoe-shi, Saitama 350-0035 (JP)
(74) Representative: IPAZ
(86) International application number: PCT/JP2023/026741
(87) International publication number: WO 2024/019141

(57) **Abstract**

[PROBLEM] To provide a formulation that is safe for ingestion over a long period of time and can improve the enterobacterial flora or can enhance the barrier function of the intestinal mucosa.

[SOLUTION] The present invention is an enteric capsule formulation for improving enterobacterial flora containing nicotinamide mononucleotide as an active ingredient.

## Description

### Technical Field

The present invention relates to an enteric capsule formulation for improving enterobacterial flora, encapsulating nicotinamide mononucleotide.

### Background Art

It is known that, in the human intestinal tract, about 1,000 kinds, 100 to 1,000 trillion of enteric bacteria live from the small intestine to the large intestine, and these enteric bacteria are deeply involved in the nutritional metabolism, the defense mechanism, the immune mechanism, and the like of human hosts. Human enteric bacteria are roughly divided into three groups: so-called good bacteria (hereinafter simply referred to as good bacteria), so-called bad bacteria (hereinafter simply referred to as bad bacteria), and intermediate bacteria (opportunistic bacteria) that neither belong to good bacteria nor bad bacteria. These bacteria are closely related to each other, and each maintains a balance and chooses a comfortable place and settles. Regarding the distribution of the enteric bacteria, there are very few bacteria that are always present from the duodenum to the upper part of the small intestine, but the number of bacteria increases toward the lower part of the small intestine, and from the lower part of the small intestine to the large intestine, the speed at which the contents pass is slow and there is almost no oxygen, and thus many bacteria that prefer such an environment (anaerobic bacteria) live. When bacteria reach the large intestine, the number of the bacteria increases rapidly and its composition becomes almost the same as that of the bacterial flora of feces. In particular, the number of obligate anaerobic bacteria such as *Bacteroides, Bifidobacterium bifidum,* and *Eubacterium* increases drastically.

Among the enteric bacteria, bad bacteria proliferate in the intestines due to Western-style diets that are mainly made up of proteins and fats, irregular lifestyles, stress, constipation, and the like. If the bad bacteria proliferate too much, it is easy to cause rough skin, a decrease in immunity, and the onset of lifestyle-related diseases. In a healthy intestine, good bacteria such as *Bifidobacterium bifidum* and *Lactobacillus* are dominant, and other bacteria are in the inferior state. When good bacteria produce lactic acid and short-chain fatty acid such as acetic acid and make the intestines acidic, they are said to suppress the proliferation of bad bacteria and activate movement of the intestine, prevent infection caused by food poisoning bacteria and pathogenic bacteria, and create an intestinal environment that suppresses the production of carcinogenic putrefactive products. Good bacteria also produce various vitamins (B₁, B₂, B₆, B₁₂, K, nicotinic acid, and the like) in the intestines. In addition, it has been reported that the substances that constitute the body of good bacteria have an effect of increasing the body's immunity and decreasing serum cholesterol. On the other hand, when good bacteria predominate, opportunistic bacteria help the production of vitamins and hormones necessary for biological activities and the activity of lipid metabolism. Conversely, when bad bacteria predominate, they produce harmful substances and contribute to activities such as weakening of the barrier function and immunity of the intestinal tract. Therefore, it is said that the golden ratio of enteric bacteria is a balance of 10% bad bacteria, 20% good bacteria, and 70% opportunistic bacteria. In the past, it was considered that the more good bacteria there were, the more preferable. However, it has been found that some bacteria that were thought to be harmful also have beneficial effects on the body, and therefore the existence of a wide variety of enteric bacteria is now considered to be preferable.

There are a wide variety of types (composition) of different enteric bacteria from person to person, and they are also said to differ depending on factors such as diet, climate, and living environments. For example, it has been revealed that the profile of the enterobacterial flora of Japanese people is dominated by, for example, *Bifidobacterium bifidum* and *Blautia,* has fewer archaebacteria, and is rich in the metabolism functions of carbohydrate or amino acid, compared to the enterobacterial flora of the United States, France, and the like. In addition, the balance of the enterobacterial flora changes with age, and the kind of enteric bacteria increases as people get older. In the elderly, the number of *Clostridium perfringens, Escherichia coli, Enterococcus faecalis, Lactobacillus,* and the like increases, and the number of *Bifidobacterium bifidum,* which was dominant when they were young, decreases. Moreover, the enterobacterial flora changes due to, for example, stress, drugs, and diseases. For example, the enteric bacteria change significantly due to the intake of medicines such as antibiotics and proton pump inhibitors (PPI) or food poisoning.

In recent years, advances in genetic analysis technologies using next-generation sequencers and the like have made it possible to comprehensively analyze the genes of enteric bacteria and their metabolic products. Therefore, the overview and the functions of the enterobacterial flora are becoming clear. As a result, it has become clear that the enterobacterial flora is closely related to diseases such as obesity, diabetes, colon cancer, arteriosclerosis, and inflammatory enteric disease, and that the enterobacterial flora of these patients is significantly different from that of healthy people. Moreover, the importance of the enterobacterial flora in maintaining human health is becoming more and more recognized than before. Therefore, disease prophylaxis methods and treatment methods aimed at maintaining a normal enterobacterial flora and correcting an abnormal enterobacterial flora are being considered. When the diversity and balance of human enteric bacteria are lost, the intestinal environment becomes disturbed, which is called dysbiosis. Dysbiosis is an abnormality in the bacterial species that constitute the enterobacterial flora, and refers to a decrease in the diversity of normally present bacterial species, an abnormal proliferation of bacterial species that are usually scarce, and an abnormal decrease in bacterial species that are usually abundant.

As the causes of dysbiosis, medicines such as antimicrobial agents and proton pump inhibitors, Western-style diets, and environmental factors have been pointed out.

It is believed that the administration of medicines such as antimicrobial agents and proton pump inhibitors disrupts the uniformity and diversity of the enterobacterial flora and results in a state of dysbiosis, resulting in the breakdown of the defense mechanism of a host.

The Western-style diets are characterized by low dietary fiber and high fat, and the low dietary fiber means that lactic acid and the short-chain fatty acid such as acetic acid produced by fermenting it are low. It has been reported that short-chain fatty acids exhibit an anti-inflammatory action, also decrease the permeability of the intestinal tract, and act so as to defend the intestinal tract. Therefore, it can be said that an insufficient intake of the dietary fiber decreases the production of short-chain fatty acids, which makes it easier to cause dysbiosis. It has also been reported that high-fat diets decrease *Bacteroidota* and induce an increase in *Firmicutes* and *Proteobacteria,* resulting in the disturbance of the balance of the enterobacterial flora. Additionally, Western-style diets contain food product additives such as artificial sweeteners, emulsifiers, and preservatives. It is suggested that some of these are related to dysbiosis. It is thought that such additives increase membrane permeability, promote the absorption of harmful substances such as lipopolysaccharides (LPS) into the gastrointestinal tract, and decrease the barrier function of the intestinal mucosa, thereby causing dysbiosis.

Regarding the environmental factors, a relevance between air pollutants and dysbiosis or inflammatory diseases has been reported.

In recent years, the number of patients with inflammatory enteric diseases such as ulcerative colitis has increased explosively, and it has been suggested that the cause is closely related to Western-style dietary patterns. As described above, the Western-style diets have been pointed out as one of the causes of dysbiosis, and it is believed that dysbiosis easily causes inflammatory enteric diseases. In other words, one of the causes of inflammatory enteric diseases, which are steadily increasing in terms of the number of patients, is thought to be the breakdown of the intestinal mucosal barrier and the disturbance of the balance of the enterobacterial flora.

The intestinal mucosal barrier is an infection defense mechanism that exists on the surface of the gastrointestinal tract, where foreign microorganisms pass with the intake of food and where many microorganisms coexist, and protects the intestinal tract tissue from these microorganisms or avoids excessive immune responses to indigenous microorganisms. This intestinal mucosal barrier is roughly divided into two types, a physical barrier and a chemical barrier, depending on their functions or properties. The physical barrier is a barrier that acts as a physical wall to prevent the invasion of microorganisms, and includes the mucus layer (mucin layer) that covers the epithelial layer of the intestinal tract, the glycocalyx that is an assembly of sugar chains existing on the surface of epithelial cells, and tight junctions that are intercellular adhesion devices. For example, in the large intestine where many enteric bacteria are present, the mucus layer, which is one of the intestinal mucosal barriers, covers the surface in a thick manner, and it is known that enteric bacteria cannot easily invade the large intestine tissue. On the other hand, the chemical barrier is a group of molecules that cause a chemical change in microorganisms through the secretion of antimicrobial peptides, and exhibit an antimicrobial activity to suppress bacterial invasion. These antimicrobial peptides include defensin family molecules and Reg3 family molecules, and these molecules are mainly produced from Paneth cells that are one of intestinal tract epithelial cells.

As described above, the intestinal tract epithelial cells have the barrier function of the intestinal mucosa that prevents the invasion of pathogens, and thus play important roles in the host defense and in maintaining homeostasis. In addition, the intestinal mucosal barrier is also important in avoiding excessive immune responses to the enteric bacteria of a host by separating the enteric bacteria from the epithelium of the intestinal tract. Therefore, when the intestinal mucosal barrier becomes abnormal, the enteric bacteria and the epithelium of the intestinal tract are spatially close to each other, which makes it easier to cause immune response to the enteric bacteria. Then, a strong immune response results in disturbance of the balance of the enterobacterial flora, leading to the onset or exacerbation of various diseases such as inflammatory enteric diseases including intestinal tract inflammation, metabolic diseases, and autoimmune diseases.

Furthermore, even if the composition of a healthy intestinal flora is disturbed, it has the ability to return to a normal, balanced state, so-called a symbiosis state, within a certain period of time. However, when dysbiosis continues for a long period of time, it is thought that this can trigger the breakdown of the intestinal tract barrier or the migration of abnormally increased bacterial toxins and metabolites into the body as a result of dysbiosis, leading to systemic chronic inflammation and the onset of inflammatory and metabolic diseases.

Thus, the intestinal mucosal barrier and the enterobacterial flora are closely related to health and diseases, and even affect the lifespan of humans. Therefore, it can be said that normally working the intestinal mucosal barrier, improving dysbiosis involved in the onset of various diseases, correcting the enterobacterial flora to a good balance found in a healthy host, and thus maintaining homeostasis will lead to the prophylaxis and treatment of diseases and are very important for the host to live in good health for a long period of time.

Conventionally, as medicines for improving the human enterobacterial flora, active probiotic formulations, which are intestinal resident bacteria and regulate an intestinal environment, have been widely known, and lactomin formulations *(Streptococcus Faecalis* and *Lactobacillus acidophilus*), *Bifidobacterium bifidum, Clostridium butyricum* (MIYAIRI), *Lactobacillus casei,* resistant *Lactobacillus,* and the like are commonly used.

Furthermore, a composition for promoting production of intestinal short-chain fatty acid, which contains fucose as an active ingredient, has been reported as a composition for promoting the production of short-chain fatty acids produced by enteric bacteria and improving the enterobacterial flora (Patent Literature 1).

As another example, an agent for improving the enterobacterial flora, which is used for prophylaxis or improvement of at least one kind selected from the group consisting of obesity, high blood pressure, and constipation, contains *Euglena* that is microalgae belonging to *Euglena,* and particularly has an action of decreasing a ratio of *Firmicutes* bacteria to *Bacteroidota* bacteria, has been reported (Patent Literature 2).

### Citation List

### Patent Literature

Patent Literature 1: JP 2019-170335 A
Patent Literature 2: JP 2019-89733 A

### Summary of Invention

### Technical Problem

Based on the above, it is believed that in order to protect the health of a host, it is important to maintain a good barrier function of the intestinal mucosa and maintain the composition of the enterobacterial flora in a healthy state. However, as mentioned above, there are various causes of the decrease in the barrier function of the intestinal mucosa and the breakdown of dysbiosis, and effective countermeasures against them have not yet been established.

Therefore, an object of the present invention is to provide a formulation, which is effective in improving the enterobacterial flora and enhancing the barrier function of the intestinal mucosa, and is safe for ingestion over a long period of time.

### Solution to Problem

As a result of diligent studies for solving the above-described problems, the present inventor found that nicotinamide mononucleotide, which is an intermediate metabolite related to biosynthesis of coenzyme nicotinamide adenine dinucleotide (NAD), is effective in improving the enterobacterial flora and enhancing the barrier function of the intestinal mucosa, and use of an enteric capsule formulation as a dosage form can solve the above problems. Then, the present inventor completed the present invention.

The present invention is as follows.
[1] An enteric capsule formulation for improving enterobacterial flora, comprising
   nicotinamide mononucleotide encapsulated therein as an active ingredient.
[2] The enteric capsule formulation for improving enterobacterial flora according to [1], wherein
   the improving enterobacterial flora is decreasing an existing ratio of *Alistipes* bacteria in the enterobacterial flora.
[3] The enteric capsule formulation for improving enterobacterial flora according to [1], wherein
   the improving enterobacterial flora is increasing an existing ratio of *Phascolarctobacterium* bacteria in the enterobacterial flora.
[4] The enteric capsule formulation for improving enterobacterial flora according to [1], wherein
   the improving enterobacterial flora is suppressing a decrease in an existing ratio of *Dorea* bacteria and/or *Lachnospira* bacteria in the enterobacterial flora.
[5] The enteric capsule formulation for improving enterobacterial flora according to [1], comprising
   enteric bacteria encapsulated therein.
[6] The enteric capsule formulation for improving enterobacterial flora according to [1], comprising
   Probiotics encapsulated therein.
[7] The enteric capsule formulation for improving enterobacterial flora according to [6], wherein
   the Probiotics is selected from strains belonging to one or more of *Acidobacteria, Actinobacteria, Firmicutes, Bacteroidota, Ascomycota, Fusobacteriota, Chlamydiae, Chloroflexi, Cyanobacteria, Deferribacteres, Deinococcus-Thermus, Fibrobacteres, Gemmatimonadota, Lentisphaerae, Planctomycetes, Spirochaetes,* Tenericutes, *Verrucomicrobia,* and *Proteobacteria.*
[8] The enteric capsule formulation for improving enterobacterial flora according to [6], wherein
   the Probiotics is selected from strains belonging to one or more of *Lactobacillus, Bacillus, Bifidobacterium, Escherichia, Saccharomyces, Enterococcus, Clostridium, Prevotella, Acidaminococcus, Fusobacterium, Veillonella, Klebsiella, Streptococcus, Megasphaera, Aerococcus, Aspergillus, Bacteroides, Lactococcus, Leuconostoc, Debaryomyces, Oenococcus, Pediococcus, Weissella,* and *Eubacterium.*
[9] The enteric capsule formulation for improving enterobacterial flora according to [6], comprising
   Prebiotics encapsulated therein.
[10] The enteric capsule formulation for improving enterobacterial flora according to [9], wherein
   the Prebiotics is one or more selected from fructo-oligosaccharide, lactosucrose, theanderose, galactooligosaccharide, lactulose, isomaltooligosaccharide, gentiooligosaccharide, trehalose, xylooligosaccharide, soybean oligosaccharide, maltitol, lactitol, reduced isomaltulose, sorbitol, and xylitol.
[11] The enteric capsule formulation for improving enterobacterial flora according to [1], wherein
   the enteric capsule formulation is used for enhancing a barrier function of the intestinal mucosa.
[12] The enteric capsule formulation for improving enterobacterial flora according to [1], wherein
   the enteric capsule formulation is a food product.
[13] The enteric capsule formulation for improving enterobacterial flora according to [1], wherein
   the enteric capsule formulation is a medicinal product.
[14] A method for improving enterobacterial flora or a method for enhancing a barrier function of intestinal mucosa, the method comprising:
   causing a target to ingest the enteric capsule formulation for improving enterobacterial flora according to any one of [1] to [13].

### Advantageous Effects of Invention

The present invention is effective in improving the enterobacterial flora and enhancing the barrier function of the intestinal mucosa, and contains nicotinamide mononucleotide, which is an intermediate metabolite related to in vivo NAD⁺ biosynthesis, as an active ingredient. Therefore, the present invention is safe and can be taken for a long period of time.

### Brief Description of Drawing

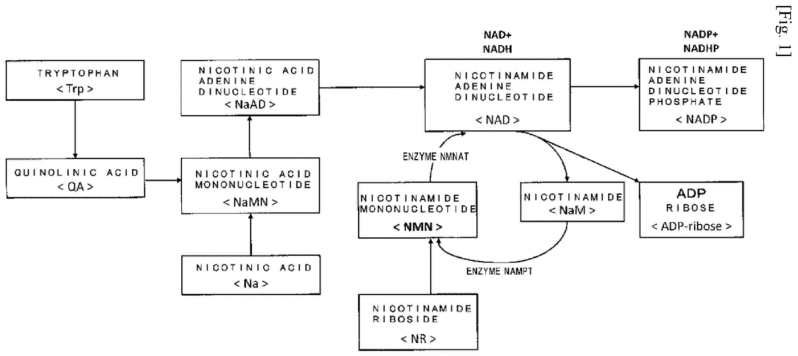

Fig. 1 is an explanatory diagram illustrating metabolic pathways related to niacin (general term for nicotinamide and nicotinic acid).

### Description of Embodiments

An enteric capsule formulation for improving enterobacterial flora according to the present invention (i.e., acid-resistant capsule, may be simply referred to as "enteric capsule formulation" hereinafter) is a capsule formulation, in which nicotinamide mononucleotide as an active ingredient and, if appropriate, other components are encapsulated in a capsule, and which is configured to be dissolved from the small intestine to the large intestine to expose the capsule contents. Therefore, the active ingredient is not inactivated by gastric acid until it reaches the small intestine or the large intestine, and the function of the capsule contents is maintained, allowing it to exhibit its function in the small intestine or the large intestine. Here, the kind of enteric capsule formulation may be any of hard capsules, soft capsules, and seamless capsules.

The detailed reason for resulting in improvement of the enterobacterial flora or enhancement of the barrier function of the intestinal mucosa by containing nicotinamide mononucleotide as an active ingredient is currently under consideration. However, as one of main reasons, it is considered that the nicotinamide mononucleotide accelerates "sirtuins" represented by NAD⁺- dependent deacetylases Sirt1 and Sirt3 and consequently activates the intestinal tract epithelial cells, to thereby improve the enterobacterial flora or enhance the barrier function of the intestinal mucosa. The following describes the present invention in detail.

The nicotinamide mononucleotide (chemical formula: C₁₁H₁₅N₂O₈P) is a compound produced in bodies of many organisms including human, and expressed with a structural formula [Chem. 1] below. The nicotinamide mononucleotide is generally referred to as NMN, and known as an intermediate metabolite involved in a biosynthesis of coenzyme NAD⁺.

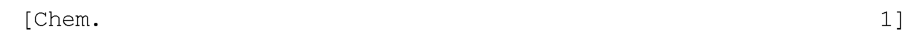

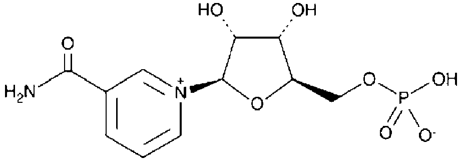

The nicotinamide mononucleotide, which is contained as an active ingredient of the enteric capsule formation, is produced in an NAD metabolic pathway by liver tissues, that is, a pathway involved in a synthesis of a nicotinamide adenine dinucleotide (NAD) from a quinolinic acid through a kynurenine pathway, in vivo. This will be specifically described with reference to Fig. 1. Fig. 1 is an explanatory drawing illustrating a metabolic pathway involved in niacin (generic term of a nicotinamide and a nicotinic acid) known as vitamin B₃. The nicotinic acid ingested through a meal is absorbed by the liver to be converted into nicotinamide, and the nicotinamide is supplied to the whole body via a blood flow. The cells each absorb the nicotinamide from the blood, and convert it into the NAD and an NADP to use them. The nicotinamide is biosynthesized also from a tryptophan.

As illustrated in Fig. 1, in vivo, when the tryptophan is a starting material, the tryptophan is converted into the quinolinic acid (QA) through the kynurenine pathway as a tryptophan metabolic pathway, and further converted into a nicotinic acid mononucleotide (NaMN). Meanwhile, when the nicotinic acid (Na) is the starting material, the nicotinic acid is directly converted into the NaMN. Afterwards, the NaMN is interconverted into the NAD, a nicotinamide (NaM), and the nicotinamide mononucleotide in a NAD cycle through a nicotinic acid adenine dinucleotide (NaAD). The nicotinamide (NaM) is converted into the nicotinamide mononucleotide by a nicotinamide phosphoribosyltransferase (NAMPT), subsequently, the nicotinamide mononucleotide is converted by a nicotinamide mononucleotide adenyltransferase (NMNAT) to generate the NAD. Note that, the nicotinamide mononucleotide is produced also from a nicotinamide riboside (NR) as an NAD intermediate metabolite.

The nicotinamide mononucleotide includes two types of an α-form and a β-form as optical isomers, and the β-form is used in the present invention. The nicotinamide mononucleotide is obtained by, for example, synthesizing a nicotinamide riboside from the nicotinamide and a ribose (see Bioorg. Med. Chem. Lett., 12, 1135-1137 (2002)), and subsequently, phosphorylating a 5-hydroxyl group of the ribose part (see Chem. Comm., 1999, 729-730). Specifically, for example, first, a reaction solution is prepared by dissolving the nicotinamide and an L-ribose tetraacetate in anhydrous acetonitrile, adding a trimethylsilyl trifluorosulfonic acid by an excessive amount under a nitrogen stream and then stirring at room temperature, and adding methanol to stop the reaction. The above-described reaction solution is poured into a column filled with activated carbon, cleaned with a distilled water, and then eluted with methanol and its product is collected. Next, for a phosphorylation reaction of the 5-hydroxyl group of the L-ribose part of this product, a reaction solution is prepared by dissolving the above-described product in a trimethoxy phosphoric acid, dropping a phosphorus oxychloride below freezing and stirring under the nitrogen stream, adding a sodium hydroxide aqueous solution to neutralize, thus stopping the reaction. A cold acetonitrile-ether solution is added to the above-described reaction solution. Afterwards, a lower layer (water phase) is passed through an anion-exchange resin to collect a reactant, and further purifies the reactant with a cation-exchange resin, thus the high-purity nicotinamide mononucleotide can be collected. The nicotinamide mononucleotide is commercially available from Oriental Yeast Co., ltd. and Bontac Bio-engineering (Shenzhen) Co., Ltd., and those commercial products can be purchased for use.

The nicotinamide mononucleotide is a purified product containing a low content of impurities, and in particular, the purity thereof is preferably 90% or more, and more preferably 95% or more. When the purity is less than 90%, a bad smell may be generated, or an action of nicotinamide mononucleotide may be attenuated, thereby possibly failing to sufficiently obtain the effect of the present invention.

The enteric capsule formulation according to the present invention is easily produced by using nicotinamide mononucleotide alone or mixing other components. The other components are not particularly limited as long as the enteric capsule formulation exhibits the effect of the present invention.

Examples of the other components include enteric bacteria. Regarding selection of specific enteric bacteria, the composition of the bacterial species of the enterobacterial flora varies depending on the person, and it is now considered that the existence of a wide variety of enteric bacteria is good for health. Based on this, it is preferable to appropriately select the strain of bacteria that is considered to lack when compared with the current enterobacterial flora based on the composition of the enterobacterial flora when each person is healthy. Widely and generally, *Bacillus subtilis var. natto, Lactobacillus,* and *Bifidobacterium bifidum* are applied. These three types of good bacteria work together to produce metabolic products (organic acids such as lactic acid and acetic acid) that keep the intestinal environment weakly acidic, and the weakly acidic environment is expected to have high health effects of preventing the proliferation of bad bacteria that are weak to acid and strengthening the barrier function of the intestinal tract.

In addition, preferable examples of the other components include Probiotics. In the present invention, Probiotics is defined as "microorganisms that have a positive effect on the human body or a product containing the microorganisms". This corresponds to so-called useful bacteria and fermented products containing the useful bacteria. In the present invention, Probiotics include not only live bacteria but also dead bacteria, and further include not only bacteria but also cell wall components such as peptidoglycan, and components such as DNA and RNA.

As specific examples of the Probiotics, it is possible to select strains belonging to one or more of *Acidobacteria, Actinobacteria, Firmicutes, Bacteroidota, Ascomycota, Fusobacteriota,* and *Proteobacteria.*

Moreover, as specific examples of the Probiotics, it is possible to select strains belonging to one or more of *Lactobacillus* (e.g., various lactobacilli), *Bacillus* (e.g., *Bacillus subtilis var. natto* and *amylolytic bacillus*), *Bifidobacterium* (e.g., various B*ifidobacterium bifidum*), *Escherichia* (e.g., *Escherichia coli*), *Saccharomyces* (e.g., *Saccharomyces cerevisiae*), *Enterococcus* (e.g., *Enterococcus faecalis* and *Enterococcus faecium*), *Clostridium* (e.g., *Clostridium butyricum, Clostridium coccoides, Clostridium leptum, Clostridium ramosum,* and *Clostridium perfringens*), *Prevotella* (e.g., *Prevotella copri*), *Acidaminococcus* (e.g., *Acidaminococcus fermentans*), *Fusobacterium* (e.g., *Fusobacterium nucleatum*), *Veillonella* (e.g., *Veillonella parvola*), *Klebsiella* (e.g., *Klebsiella pneumoniae*), *Streptococcus* (e.g., *Streptococcus thermophilus* and *Streptococcus faecalis*), *Megasphaera* (e.g., *Megasphaera elsdenii*), *Aerococcus* (e.g., *Aerococcus viridans*), *Aspergillus* (e.g., *Aspergillus oryzae*), *Bacteroides* (e.g., *Bacteroides plebeius*), *Lactococcus* (e.g., *Lactococcus lactis*), *Leuconostoc* (e.g., *Leuconostoc mesenteroides*), *Debaryomyces* (e.g., *Debaryomyces tamarii*), *Oenococcus* (e.g., *Oenococcus oeni*), *Pediococcus* (e.g., *Pediococcus acidilactici*), *Weissella* (e.g., *Weissella cibaria*), and *Eubacterium* (e.g., *Eubacterium limosum*).

In particular, when Probiotics are contained, it is preferable to also contain Probiotics in combination with Prebiotics. When Probiotics and Prebiotics are taken at the same time (synbiotics), the intestinal environment can be more effectively improved. Prebiotics are generally defined as "indigestible food product ingredient that has a beneficial effect on a host by either proliferating good bacteria normally present in the large intestine or inhibiting the proliferation of harmful bacteria." The "beneficial effect" includes a wide range of effects, such as infection prevention, immune response regulation, blood pressure and blood sugar regulation, promotion of absorption of minerals, skin health, and stress relief.

Specific examples of the Prebiotics include one or more selected from fructo-oligosaccharide, lactosucrose, theanderose, galactooligosaccharide, lactulose, isomaltooligosaccharide, gentiooligosaccharide, trehalose, xylooligosaccharide, soybean oligosaccharide, maltitol, lactitol, reduced isomaltulose, sorbitol, and xylitol. It is preferable to select one suitable for enhancing proliferation of Probiotics used.

The enteric capsule formulation according to the present invention can be used for improving the enterobacterial flora or for enhancing the barrier function of the intestinal mucosa. In the present invention, "improving enterobacterial flora" means not only returning to or approaching the diversity or the balance of healthy enteric bacteria in the enterobacterial flora when compared with the enterobacterial flora of a healthy host, but also means prevention or delay of deterioration of the state of the enterobacterial flora in the broad sense. In addition, the "enhancing the barrier function of the intestinal mucosa" means not only enhancing the barrier function of the intestinal mucosa in the narrow sense (returning to or approaching the barrier function of the intestinal mucosa of a healthy host) when compared with the barrier function of the intestinal mucosa of a healthy host, but also means prevention or delay of deterioration of the barrier function of the intestinal mucosa in the broad sense.

In the present invention, specific examples of the improvement of the enterobacterial flora include decreasing the existing ratio of *Alistipes* bacteria in the enterobacterial flora, increasing the existing ratio of *Phascolarctobacterium* bacteria, and inhibiting a decrease in the existing ratio of *Dorea* bacteria and/or *Lachnospira* bacteria.

The aspects of the enteric capsule formulation according to the present invention are roughly classified into an enteric capsule formulation in which capsule contents (enteric granules) coated with an enteric polymer are encapsulated in a capsule, and an enteric capsule formulation in which capsule contents are encapsulated in an enteric capsule coated with an enteric polymer, i.e., a capsule that dissolves in the small intestine or the large intestine. Note that an enteric capsule formulation, in which enteric granules are encapsulated in an enteric capsule coated with an enteric polymer, is also included.

In the former enteric capsule formulation, examples of the enteric polymer include a substance that exhibits good solubility in an aqueous solvent having a neutral to alkaline pH value supposed to be in the intestinal environment. Specific examples thereof include methacrylic acid-based polymers (e.g., methacrylic acid copolymer L, methacrylic acid copolymer LD, and methacrylic acid copolymer S), cellulose-based polymers (e.g., carboxy methyl ethyl cellulose, hypromellose phthalate, and hypromellose acetate succinate), and vinyl-based polymers (e.g., polyvinyl acetate phthalate and polyvinyl butyrate phthalate), which dissolve in the pH range of about 5 to 12.0.

The latter enteric capsule formulation includes both an enteric capsule formulation in which the surface of the capsule is subjected to acid-resistant coating with an enteric polymer, and an enteric capsule formulation in which the surface of the capsule itself is acid-resistant and therefore does not need acid-resistant coating with an enteric polymer.

The former enteric capsule formulation, in which the capsule contents (enteric granules) coated with the enteric polymer are encapsulated in a capsule, is produced by, for example, the following method. First, as a method for obtaining enteric granules by coating capsule contents with an enteric polymer, commonly used fine particle coating methods such as a fluidized bed granulation method and a fine particle coating method can be used. For example, in the fine particle coating method, a coating liquid that contains, for example, an enteric polymer is sprayed and dried onto a particulate raw material charged into a fluidized bed granulation apparatus so as to be coated. Then, enteric granules can be obtained. The coating amount may be appropriately set so that a function of releasing the particulate raw material for the first time in the small intestine or the large intestine is achieved. Then, the enteric granules are encapsulated in a gelatin capsule, a normal capsule formed of, for example, a cellulose derivative or starch, or an enteric capsule, together with additives, if necessary, to produce the enteric capsule formulation.

The latter enteric capsule formulation, in which capsule contents are encapsulated in an enteric capsule, is produced by, for example, the following method. The enteric polymer is coated on a gelatine capsule that is not enteric, or a normal capsule that contains a cellulose derivative or the like, with a fluidized bed-type coating apparatus, a rotary pan-type coating apparatus, or the like, to thereby obtain an enteric capsule. The coating amount may be appropriately set so that a function of releasing the capsule contents for the first time in the small intestine or the large intestine is achieved. Then, the capsule contents are encapsulated in the enteric capsule, to produce the enteric capsule formulation. Note that a commercially available product as an enteric capsule can be used as long as a function of releasing the capsule contents for the first time in the small intestine or the large intestine is achieved.

In the enteric capsule formulation according to the present invention, the respective amounts of nicotinamide mononucleotide, Probiotics, and Prebiotics to be blended may be appropriately determined taking into consideration the number of times of daily intake and the like, with reference to the respective daily intakes described below.

The enteric capsule formulation according to the present invention can be used as a food product or a medicinal product. When the enteric capsule formulation according to the present invention is daily ingested in the form of a food product, the effect of the present invention can be provided repeatedly, which is particularly effective in improving the balance or diversity of the enterobacterial flora. The type of the food product as the target of the present invention is not particularly limited, and the target includes a functional food, a food for specified health use, a dietary supplement, a food additive, a feed, a care food, a diet therapy food, a therapeutic diet, a diet food, and similar food product in addition to general food products.

The intake of the food product is different depending on the type of the food product, age, sex, and weight of a target that takes the food product, the expected effect, and the like. However, the daily intake per adult of the nicotinamide mononucleotide contained in the food product is generally 1 mg to 500 mg, preferably 5 mg to 400 mg, and more preferably 50 mg to 300 mg. Less than 1 mg possibly fails to provide the effect of the present invention, while more than 500 mg merely provides almost similar effect but causes economic disadvantage. The compounding ratio of the nicotinamide mononucleotide in the food product can be appropriately set in a range of 100% or less, relative to the total weight of the food product.

The intake of the Probiotics contained in the food product is only required to be appropriately determined depending on the kind of the strains and the like. The daily intake per adult can be, for example, 10⁷ cfu to 10¹¹ cfu, or 10⁹ cfu to 10¹⁰ cfu.

The intake of the Prebiotics contained in the food product is preferably such an amount suitable for promoting proliferation of Probiotics used. The daily intake per adult is generally in the range of 1 mg to 20 g, and is only required to be appropriately determined depending on the kind of the strains and the like.

The food product is safe and is not found to have particular side effects, so it can be taken for a long period of time. It is applicable not only to the elderly but also to young people.

On the other hand, the enteric capsule formulation according to the present invention can be orally administered as a medicinal product (including quasi-drugs) for improving the enterobacterial flora or enhancing the barrier function of the intestinal mucosa in the pharmaceutical field. In addition, the medicinal product can be appropriately mixed with known pharmaceutical additives that are pharmaceutically acceptable, taking into consideration physicochemical properties, biological properties, etc.

A dose of the medicinal product cannot be equally specified because it differs depending on, for example, age, sex, and weight of a target that takes the food product, the expected effect, the symptom, and the number of times of the administration. However, as the dose of the medicinal product, the daily amount per adult of the nicotinamide mononucleotide to be administered can be generally 1 mg to 500 mg, preferably 5 mg to 400 mg, and more preferably 50 mg to 300 mg. Less than 1 mg possibly fails to provide the effect of the present invention, while more than 500 mg merely provides almost similar effect but causes economic disadvantage. The compounding ratio of the nicotinamide mononucleotide in the medicinal product can be appropriately set in accordance with the dosage form and the dose of the medicinal product, and the like.

The dose of Probiotics contained in the medicinal product is only required to be appropriately determined depending on the kind of the strains and the like. The daily dose per adult can be, for example, 10⁷ cfu to 10¹¹ cfu, or 10⁹ cfu to 10¹⁰ cfu.

The dose of Prebiotics contained in the medical product is preferably such an amount suitable for promoting proliferation of Probiotics used. The daily dose per adult is generally in the range of 1 mg to 20 g, and is only required to be appropriately determined depending on the kind of the strains and the like.

The number of times of the administration of the medicinal product can be appropriately set in accordance with, for example, the age, weight, and symptom of the administration target and the dose per administration of the medicinal product. The number of times of the administration of the medicinal product per day can be exemplified by once to three times.

The present invention further provides a method for improving the enterobacterial flora or a method for enhancing the barrier function of the intestinal mucosa, in which the enteric capsule formulation is administered to a subject. The target of the ingestion is preferably a mammal such as a human, a mouse, a rat, a rabbit, a dog, a cat, cattle, a horse, a pig, and a monkey, and especially a human is preferable. In the method, the intake of the enteric capsule formulation, the number of daily intakes, and the like are as described for the enteric capsule formulation. The enteric capsule formulation can be ingested anytime in any case, and can be ingested by the target over a long period of time.

### Examples

The present invention will be described in detail below with Examples and Comparative Examples, but the present invention is not limited to the following Examples.

### [Example]

Fifty-eight ordinary male and female subjects aged 45 years or older and younger than 65 years were divided into Group I subjects (the intake group of an NMN-containing enteric capsule formulation (capsule formulation 1): 30 subjects) and Group II subjects (the intake group of placebo (capsule formulation 2): 28 subjects), and each capsule formulation was orally taken for four weeks in a placebo-controlled double-blind comparative test. The subjects of Group I took two capsules of the capsule formulation 1 per day, and the subjects of Group II took two capsules of the capsule formulation 2 per day. As the capsule formulation 1 and the capsule formulation 2, the enteric capsule formulations containing the following components were used.
Capsule formulation 1: 150 mg of NMN, 50 mg of starch, and 60 mg of hydroxypropyl methylcellulose (per capsule)
Capsule formulation 2: 200 mg of starch and 60 mg of hydroxypropyl methylcellulose (per capsule)

Feces were collected at the start of intake and 4 weeks after the start of intake, and changes in the enterobacterial flora of the subjects due to administration of the enteric capsule formulation of the present invention were analyzed. Analysis was performed on the bacteria belonging to the genera, and *Alistipes, Phascolarctobacterium, Dorea,* and *Lachnospira* were analyzed as bacteria that exhibit an existing ratio of more than 0.1% in the enterobacterial flora, and their relative existing ratios relative to the total bacterial species were examined. Analysis of the enterobacterial flora was performed using a next-generation sequencer. Analysis of the bacterial flora in the feces was outsourced to Healthcare Systems Co., Ltd.

### [Results and discussion]

Table 1 shows the mean ± SD of the existing ratio of each of the above bacterial species for Group I subjects (NMN) and Group II subjects (placebo). Table 2 shows a difference between before the start of intake and after 4 weeks of intake, pre-post comparison between before the start of intake and after 4 weeks of intake for each group, and intergroup comparison between Group I and Group II for before the start of intake, after 4 weeks of intake, and the difference between before the start of intake and after 4 weeks of intake. Note that the values of the pre-post comparison were determined by the Wilcoxon signed-rank test. The values of the intergroup comparison were determined by the Wilcoxon-Mann-Whitney Test.

As can be seen from Tables 1 and 2, a change in *Alistipes* before and after intake of the enteric capsule formulation was significantly higher in Group II than in Group I, and a significant increase was observed in Group II before and after intake of the enteric capsule formulation. *Alistipes* are enteric bacteria that have been reported to be involved in various diseases, and have been reported to act pathogenically on hypertension, cardiovascular diseases, psychiatric diseases, and colon cancer. Therefore, it is considered that the existence of *Alistipes* results in an effect that is not beneficial to humans. In addition, it was confirmed that the intake of the NMN-containing enteric capsule formulation suppresses *Alistipes,* and provides an effect of decreasing *Alistipes.*

A change in *Phascolarctobacterium* before and after intake of the enteric capsule formulation was significantly higher in Group I than in Group II, and a significant increase was observed in Group I before and after intake of the enteric capsule formulation. *Phascolarctobacterium* are bacteria known to use succinic acid as the only fermentation substrate, and it has been reported that excessive succinic acid in the intestine induces diarrhea. Therefore, it is considered that the existence of *Phascolarctobacterium* using succinic acid provides a beneficial effect on humans. In addition, it was confirmed that the intake of the NMN-containing enteric capsule formulation provides an effect of increasing *Phascolarctobacterium.*

Regarding a change in *Dorea* and *Lachnospira* before and after intake of the enteric capsule formulation, there was no significant difference between the groups, but a significant decrease was observed regarding a change before and after intake of the enteric capsule formulation in group II. It has been reported that *Dorea* is positively correlated with the amount of human defensin HD5 in the feces, which is an intestinal tract immunity marker in humans and an antimicrobial peptide that decreases with age, and that the relative proportion increases in the low-sugar therapy that is proven to improve the symptoms of the irritable bowel syndrome. Therefore, it is considered that the existence of *Dorea* provides a beneficial effect on humans. *Lachnospira* are known as bacteria that produce butyric acid, and butyric acid has beneficial effects in the intestines such as an anti-inflammatory effect and an effect of absorbing minerals. Therefore, it is considered that the existence of *Lachnospira* provides a beneficial effect on humans. In addition, it was confirmed that the intake of the NMN-containing enteric capsule formulation provides an effect of suppressing a decrease in *Dorea* and *Lachnospira* as seen in Group II.

As described above, it was found that the intake of the NMN-containing enteric capsule formulation has a preferable effect on *Alistipes, Phascolarctobacterium, Dorea,* and *Lachnospira,* compared to the group of Placebo control, and is effective in improving the enterobacterial flora.

**[Table 1]**

| | 0 w | | 4 w | |
|---|---|---|---|---|
| Group | NMN | Placebo | NMN | Placebo |
| Number of N | 30 | 28 | 30 | 28 |
| *Alistipes* | 2.13±1.89 | 2.7312.11 | 1.95±2.01 | 4.10±4.48 |
| *Phascolarctobacterium* | 0.57±0.69 | 0.89±0.71 | 0.90±1.02 | 1.00±1.32 |
| *Dorea* | 0.86±1.10 | 0.85±0.89 | 0.69±0.99 | 0.54±0.77 |
| *Lachnospira* | 1.16±1.37 | 1.54±1. 63 | 0.94±1.11 | 0.90±0.98 |

**[Table 2]**

| | Difference 4 w - 0 w | | Pre-post comparison | | Intergroup comparison | | |
|---|---|---|---|---|---|---|---|
| Group | NMN | Placebo | NMN | Placebo | 0 w | 4 w | Difference 4 w - 0 w |
| *Alistipes* | -0.18±1.51 | 1.37±3.26 | 0.527 | 0.021 | 0.277 | 0.052 | 0.033 |
| *Phascolarctobacterium* | 0.33±0.73 | 0.11±1.05 | 0.009 | 0.758 | 0.031 | 0.793 | 0.018 |
| *Dorea* | -0.18±0.86 | -0.31±0.60 | 0.306 | 0.012 | 0.692 | 0.609 | 0.209 |
| *Lachnospira* | -0.22±1.10 | -0.64±1.51 | 0.231 | 0.004 | 0.306 | 0.852 | 0.269 |

## Claims

1. An enteric capsule formulation for improving enterobacterial flora, comprising
nicotinamide mononucleotide encapsulated therein as an active ingredient.

2. The enteric capsule formulation for improving enterobacterial flora according to claim 1, wherein
the improving enterobacterial flora is decreasing an existing ratio of *Alistipes* bacteria in the enterobacterial flora.

3. The enteric capsule formulation for improving enterobacterial flora according to claim 1, wherein
the improving enterobacterial flora is increasing an existing ratio of *Phascolarctobacterium* bacteria in the enterobacterial flora.

4. The enteric capsule formulation for improving enterobacterial flora according to claim 1, wherein
the improving enterobacterial flora is suppressing a decrease in an existing ratio of *Dorea* bacteria and/or *Lachnospira* bacteria in the enterobacterial flora.

5. The enteric capsule formulation for improving enterobacterial flora according to claim 1, comprising
enteric bacteria encapsulated therein.

6. The enteric capsule formulation for improving enterobacterial flora according to claim 1, comprising
Probiotics encapsulated therein.

7. The enteric capsule formulation for improving enterobacterial flora according to claim 6, wherein
the Probiotics is selected from strains belonging to one or more of *Acidobacteria, Actinobacteria, Firmicutes, Bacteroidota, Ascomycota, Fusobacteriota, Chlamydiae, Chloroflexi, Cyanobacteria, Deferribacteres, Deinococcus-Thermus, Fibrobacteres, Gemmatimonadota, Lentisphaerae, Planctomycetes, Spirochaetes,* Tenericutes, *Verrucomicrobia,* and *Proteobacteria.*

8. The enteric capsule formulation for improving enterobacterial flora according to claim 6, wherein
the Probiotics is selected from strains belonging to one or more of *Lactobacillus, Bacillus, Bifidobacterium, Escherichia, Saccharomyces, Enterococcus, Clostridium, Prevotella, Acidaminococcus, Fusobacterium, Veillonella, Klebsiella, Streptococcus, Megasphaera, Aerococcus, Aspergillus, Bacteroides, Lactococcus, Leuconostoc, Debaryomyces, Oenococcus, Pediococcus, Weissella,* and *Eubacterium.*

9. The enteric capsule formulation for improving enterobacterial flora according to claim 6, comprising
Prebiotics encapsulated therein.

10. The enteric capsule formulation for improving enterobacterial flora according to claim 9, wherein
the Prebiotics is one or more selected from fructo-oligosaccharide, lactosucrose, theanderose, galactooligosaccharide, lactulose, isomaltooligosaccharide, gentiooligosaccharide, trehalose, xylooligosaccharide, soybean oligosaccharide, maltitol, lactitol, reduced isomaltulose, sorbitol, and xylitol.

11. The enteric capsule formulation for improving enterobacterial flora according to claim 1, wherein
the enteric capsule formulation is used for enhancing a barrier function of the intestinal mucosa.

12. The enteric capsule formulation for improving enterobacterial flora according to claim 1, wherein
the enteric capsule formulation is a food product.

13. The enteric capsule formulation for improving enterobacterial flora according to claim 1, wherein
the enteric capsule formulation is a medicinal product.

14. A method for improving enterobacterial flora or a method for enhancing a barrier function of intestinal mucosa, the method comprising:
causing a target to ingest the enteric capsule formulation for improving enterobacterial flora according to any one of claims 1 to 13.
